# EUROPÄISCHE PATENTSCHRIFT

(11) **EP 4 481 363 B1**
(45) Veröffentlichungstag und Bekanntmachung des Hinweises auf die Patenterteilung: **10.06.2026**
(21) Anmeldenummer: 24183392.0
(22) Anmeldetag: 20.06.2024
(51) Int. Cl.: G01N 1/22, G01N 33/497

(54) **GASPROBENSAMMLER**
GAS SAMPLE COLLECTOR
COLLECTEUR D'ÉCHANTILLONS DE GAZ

(30) Priorität: 23.06.2023 DE 102023116568; 25.09.2023 DE 102023125890
(43) Veröffentlichungstag der Anmeldung: 25.12.2024
(73) Patentinhaber: Albert-Ludwigs-Universität Freiburg, 79098 Freiburg (DE)
(72) Erfinder: Wallrabe, Ulrike, 79089 Freiburg (DE); Frey, Yasmina, 79089 Freiburg (DE); Werner, Christiane, 79089 Freiburg (DE)
(74) Vertreter: Rösler, Uwe

(56) Entgegenhaltungen:
- CN-A- 1 677 074
- CN-A- 109 030 120
- CN-U- 210 221 600
- DE-A1- 3 530 669
- US-A- 5 503 005

## Beschreibung

### Technisches Gebiet

Die Erfindung bezieht sich auf einen Gasprobensammler zum Erfassen von Gaswechselvorgängen an einem Pflanzenblatt mit einer Probenkammer sowie ein diesbezügliches Verfahren zum Sammeln derartiger Gasproben.

Pflanzen und insbesondere Bäume können miteinander kommunizieren und sich sogar gegenseitig vor Gefahren warnen, bspw. vor Schädlingsbefall, indem sie über die Spaltöffnungen, die sich bei den meisten Gehölzen an der Unterseite ihrer Blätter befinden, eine Vielzahl von Gasen freisetzen. Die Zusammensetzung und Menge der abgegebenen Gase sind stark von den Umweltbedingungen abhängig und variieren je nach Stress der Pflanze. Zu den emittierten Gasen gehören Sauerstoff, Wasserdampf, Kohlendioxid sowie eine große Vielzahl von flüchtigen organischen Verbindungen (volatile organic compounds, kurz: VOCs). VOCs haben niedrige molare Flussraten (nmol/s) und sind daher schwer zu erkennen und zu messen.

### Stand der Technik

Die Druckschrift DE 195 22 444 C2 offenbart eine Gaswechselkammer zur Erfassung von Gaswechselvorgängen, wie sie beispielsweise bei der Photosynthese, d.h. bei der Atmung von Pflanzen auftreten. Die bekannte Gaswechselkammer weist einen Träger auf, in dem ein zu untersuchendes Pflanzenblatt einspannbar ist. Einseitig schließt das Pflanzenblatt eine gasdurchströmte Messkammer ab, in der wenigstens ein Sensor angebracht ist. Unmittelbar über der Blattoberfläche ist eine Beleuchtungseinheit angeordnet, um das Blatt mit einer definierten Lichtwellenlänge und Lichtintensität zu bestrahlen.

Ein anderer Ansatz zur Erfassung von Gaswechselvorgängen an einem Pflanzenblatt wird in dem Artikel von Haberstroh, S. et al. "Natural Carbon Isotope Composition Distinguishes Compound Groups of Biogenic Volatile Organic Compounds (BVOC) in Two Mediterranean Woody Species" in Frontiers in Plant Sience vol. 9, p. 1071, 2018 beschrieben. Hierbei dient ein Schlauchbeutel, der möglichst gasdicht über einen Ast, der eine Vielzahl von Blättern trägt, gezogen und fixiert wird. Der Schlauchbeutel ist mit einer Schlauchzu- und -ableitung verbunden, über die entsprechende Messgase aus dem Schlauchbeutelvolumen entnommen werden können. Eine mit dieser bekannten Anordnung vergleichbare Vorrichtung ist in der Druckschrift KR 10 2022 0 057 810 A offenbart, die zum Sammeln von Terpenen im Wege von Ausdunstungen einer Zypresse einen Sammelbeutel vorsieht, der über einen Astbereich einer Zypresse möglichst luftdicht angebracht ist. Mit dem von dem Sammelbeutel umschlossenen Beutelvolumen in der Größe von 20 I bis 100 I sind eine Gaszu- und Gasabführleitung verbunden, die i.V.m, einer Strömungseinheit für eine Gasdurchströmung durch das Beutelvolumen sorgt. Eine an der Gasabführungsleitung nachfolgend angeordnete Messeinheit erfasst die im abgeführten Gasstrom enthaltenen Terpene.

Die Druckschrift CN 2 10 269 312 U beschreibt eine Untersuchungsanordnung, bei der ein einzelnes Blatt in einer sogenannten Blattkammer vollständig eingeschlossen wird, an die eine Gaszu- und Gasabführungsleitung anschließt, die mit einer Analyseeinheit verbunden ist. Während der Gasuntersuchung ist das Blatt von der Blattkammer umfasst und erfährt somit einen maximalen Stress.

Die Druckschrift WO 2021/011447 A1 offenbart eine Gasprobenentnahme von einem Blatt, bei der ein Smartphone zum Einsatz kommt, das über eine Sensorschnittstelle verschiedene volatile organische Komponenten zu messen vermag. Hierzu saugt eine Anordnung die von einem Blatt evaporierten Gase an, in der die Gase einer Art chromathographischen Analyse unterzogen werden, die mittels der Fotoeinheit des Smartphones erfasst und dokumentiert wird.

Die Druckschrift CN 210221600 U beschreibt einen Gasprobensammler für pflanzliche Materialien z.B. Laubblätter, das mittels einer Beleuchtungseinheit zur Photosynthese angeregt wird und die dadurch initiierte Gasfreisetzung gesammelt wird. Die Vorrichtung weist hierzu zwei Gasprobensammelkammern auf, die auf der Blattober- und -unterseite zur Anlage gebracht und von einem Gasstrom durchströmt werden. Der abströmende Gasstrom wird in einem Sammelbehältnis gesammelt. Die Druckschrift US 5,503,005 A beschreibt eine Vorrichtung zur beidseitigen Anbringung an ein Pflanzenblatt unter Ausbildung jeweils einer Durchströmungskammer, die einseitig jeweils von einer Blattoberfläche begrenzt ist, und die zum Zwecke des Abwaschens von auf der Blattoberfläche anhaftenden Sprühaufträgen mit einer Flüssigkeit durchströmt wird, die im Anschluss einer Analyse unterzogen wird.

Die Druckschrift CN 1677074 A beschreibt eine auf einer technischen Oberfläche vakuumdicht anbringbare Messkammer zur Sammlung von an der Oberfläche anhaftenden organischen flüchtigen Stoffen.

Die Druckschrift CN 109030120 A offenbart ein Erfassungsgerät für brennbare Gase, die durch den Pflanzenstoffwechsel freigesetzt werden. Hierzu wird über eine Topfpflanze eine Art Käseglocke angeordnet, an deren oberen Ende eine Gassammel und -detektionsvorrichtung angebracht ist.

### Darstellung der Erfindung

Der Erfindung liegt die Aufgabe zugrunde, einen Gasprobensammler zum Erfassen von Gaswechselvorgängen an einem Pflanzenblatt mit einer Probenkammer derart weiterzubilden, so dass der in einem Pflanzenblatt stattfindende natürliche Gasaustausch so geringfügig wie nur möglich gestört wird. Überdies soll die Gasprobensammlung pflanzenblattselektiv durchgeführt werden können, ohne dabei das zu untersuchende Pflanzenblatt zu schädigen. Ferner soll es möglich sein, den Gasprobensammler möglichst klein, leichtgewichtig und kostengünstig auszubilden, so dass nach Anbringen des Gasprobensammlers an einem Pflanzenblatt dieser von dem mit der Pflanze verbundenen Pflanzenblatt getragen wird, d.h. die durch den Gasprobensammler auf das Pflanzenblatt wirkende Last sollte signifikant kleiner sein als die maximale Haltekraft, mit der ein Pflanzenblatt mit der Pflanze verbunden ist. Zudem soll die Möglichkeit eröffnet werden, eine Pflanze mit einer großen Vielzahl von Gasprobensammlern an ihren Blättern zu bestücken, um so Aufschluss auf den Gasprobenaustausch einer ganzen Pflanze über ihre Blätter zu erhalten.

Die Lösung der der Erfindung zugrunde liegenden Aufgabe ist im Anspruch 1 angegeben. Gegenstand des Anspruches 14 ist ein Verfahren zur Gasprobensammlung für ein Erfassen von Gaswechselvorgängen an einem Pflanzenblatt. Den Erfindungsgedanken in vorteilhafter Weise weiterbildende Merkmale sind Gegenstand der Unteransprüche sowie der weiteren Beschreibung, insbesondere unter Bezugnahme auf die illustrierten Ausführungsbeispiele zu entnehmen.

Ein lösungsgemäßer Gasprobensammler zum Erfassen von Gaswechselvorgängen an einem Pflanzenblatt mit einer Probenkammer zeichnet sich dadurch aus, dass die Probenkammer eine Probenkammerwand besitzt, in der eine Zu- und eine Abströmöffnung angeordnet ist und die einen Raumbereich begrenzt, der über eine von einem unterbrechungsfrei umlaufenden Probenkammerrand umgebende Probenkammeröffnung frei zugänglich ist. Längs des Probenkammerrandes ist eine Fügeeinheit angebracht, die geeignet ausgebildet ist, den Probenkammerrand fluiddicht an eine Oberfläche eines Pflanzenblattes derart lösbar fest zu fügen, dass im gefügten Zustand das Pflanzenblatt den Raumbereich gemeinsam mit der Probenkammerwand begrenzt.

Die der Erfindung zugrunde liegende Idee sieht eine möglichst minimalinvasive Kontaktierung und Beeinträchtigung eines "lebenden" Pflanzenblattes durch den Kontakt mit der Probenkammer vor, d.h. auch nach Entfernen des Gasprobensammlers bleibt das Pflanzenblatt unversehrt mit der Pflanze verbunden und erleidet keinerlei Schäden. Der lösungsgemäße Gasprobensammler ist nach Größe und Gewicht derart ausgewählt, dass er während der Gassammlung selbsttragend am Blatt anhaftet und dieses während der Gassammlung geringstmöglich beeinflusst bzw. beeinträchtigt, um den auf das Blatt einwirkenden Stress so gering wie möglich zu halten.

Gaswechselvorgänge finden an einem Pflanzenblatt zum überwiegenden Anteil an der Unterseite eines Pflanzenblattes statt, zumal sich dort die für den Gasaustausch verantwortlichen Spaltöffnungen (Stomata) befinden, ist die lösungsgemäße Probenkammer derart ausgebildet, so dass sie an der Unterseite eines Pflanzenblattes schonend und fluiddicht angebracht werden kann, so dass die übrige Oberfläche, insbesondere die Blattoberseite des Pflanzenblattes, frei und durch den Gasprobensammler weitgehend unbeeinträchtigt bleibt. Der größte Teil der Blattoberfläche ist somit den natürlichen Umweltbedingungen, wie beispielsweise Wind, Sonnenlicht, Regen, Bodengase etc. ausgesetzt, so dass das Pflanzenblatt während der Gasprobensammlung unbeeinträchtigt insbesondere im Hinblick auf dessen Photosynthese-Aktivität bleibt.

Die Probenkammer selbst ist in einer bevorzugten Ausführungsform halbschalenförmig bzw. kuppelförmig ausgebildet und weist einen die Schalen- bzw. Kuppelform vollständig umlaufenden Probenkammerrand auf, der vorzugsweise eine virtuelle Ebene aufspannt. Um die Probenkammer an der Unterseite eines Pflanzenblattes zu befestigen, ohne das Pflanzenblatt mechanisch zu schädigen, ist ein Fügemittel vorgesehen, das vorzugsweise in Art einer eine ringförmigen, elastische Flächendichtung ausgebildet ist und sich zumindest im gefügten Zustand längs des Probenkammerrandes unterbrechungsfrei erstreckt und eine mit der Probenkammeröffnung möglichst großflächig überlappende Öffnung besitzt. Die vorzugsweise aus einem transparenten Silikonmaterial gefertigte elastische Flächendichtung sorgt zum einen dafür, dass die Probenkammer fluiddicht längs ihres gesamten Probenkammerrandes mit der elastischen Flächendichtung verbunden ist, zum anderen liegt sie über ihre kreisringförmige Kontaktfläche fluiddicht an der Unterseite des Pflanzenblattes an und vermag die für die Befestigung der Probenkammer am Pflanzenblatt erforderlichen Halte- bzw. Kontaktkräfte möglichst großflächig und gleichmäßig an der Blattunterseite zu verteilen.

Für die Erzeugung der Halte- bzw. Kontaktkräfte sieht der Gasprobensammler ein Fügemittel mit wenigstens zwei Magneteinheiten vor, von denen eine erste Magneteinheit fest oder lösbar fest mit der Probenkammer verbunden oder verbindbar ist und eine zweite Magneteinheit als separat handhabbare Einheit ausgebildet ist. Die erste Magneteinheit kann, wie erwähnt, entweder Teil der Probenkammer sein, d.h. untrennbar mit dieser bspw. längs des Probenkammerrandes verlaufend angeordnet, oder innerhalb der elastischen Flächendichtung gefügt oder herausnehmbar fügbar ausgebildet sein. Ferner ist der Probenkammerrand in die elastische Flächendichtung fluiddicht gefügt oder fügbar ausgebildet. Vorzugsweise bietet es sich an, die an einer dem Pflanzenblatt gegenüberliegenden Oberfläche der elastischen Flächendichtung mit einer nutförmigen, an die Geometrie und Dimension des Probenkammerrandes entsprechend gegenkonturiert angepasste Ausnehmung vorzusehen, in die der Probenkammerrand fluiddicht unter Ausbildung einer Form-Kraftschlussverbindung einsetzbar ist.

Die zweite Magneteinheit entspricht vorzugsweise in Form und Größe der ersten Magneteinheit und ist im gefügten Zustand deckungsgleich zur ersten Magneteinheit an der, der Probenkammer gegenüberliegende Oberfläche des Pflanzenblattes anbringbar. Vorzugsweise ist zwischen der zweiten Magneteinheit und dem zu schützenden Pflanzenblatt ein elastisches, lichttransparentes Flächenelement angeordnet, um einerseits die Kontaktkräfte möglichst homogen zu verteilen, andererseits den Abschattungseffekt auf der Oberseite des Pflanzenblattes möglichst gering zu halten, so dass das Pflanzenblatt dessen Photosynthese-Aktivität trotz Anbringung des Gasprobensammlers möglichst uneingeschränkt fortsetzen kann. Besonders vorteilhaft ist eine lichttransparente Ausbildung der ersten und vor allem zweiten Magneteinheit, bspw. durch Einsatz von lichttransparenten magnetischen Materialien.

Neben der dem Pflanzenblatt zugewandten Probenkammeröffnung sind in der Probenkammerwand darüber hinaus eine Zu- und eine Abströmöffnung vorgesehen, die dem fluiddichten Anschluss einer Gaszuführungsleitung sowie einer Gasabführungsleitung dienen. Der Gasprobensammler samt der für dessen Funktion erforderlichen Gaszuführungsleitung und Gasabführungsleitung sind minimalgewichtig ausgeführt und für eine unmittelbare Befestigung am Pflanzenblatt sowie längs des das zu untersuchende Pflanzenblatt tragenden Astes ausgebildet. So beträgt das Gewicht des Gasprobensammlers, d.h. ohne die Gaszuführungs- und Gasabführungsleitung, wenige einzelne Gramm bis maximal ca. 20 Gramm, je nach Abhängigkeit von Blattgröße und Blattstabilität sowie der damit verbundenen mechanischen Traglast des Pflanzenblattes. Auch die Dimension und Form der Probenkammer ist grundsätzlich an die Geometrie und Stabilität des jeweils zu untersuchenden Pflanzenblattes anpassbar. Beispielsweise sind Probenkammern zur Untersuchung an Ahorn-Blättern größer, stabiler und schwerer auszubilden als jene bspw. für Akazien-Blätter.

Allen lösungsgemäßen Gasprobensammlern liegt die Maxime zugrunde, dass sie selbsttragend am Blatt, d.h. vorzugsweise an der Blattunterseite, zu befestigen sind, ohne das Blatt während und auch nach der Gasprobensammlung in seiner biologischen Funktion zu beeinträchtigen.

Nicht notwendigerweise, jedoch in vorteilhafter Weise ist die Probenkammer rotationssymmetrisch zu einer orthogonal zu der vom Probenkammerrand aufgespannten virtuellen Ebene ausgebildet und erhebt sich über diese kuppelartig bzw. domförmig. Vorzugsweise ist die Zuströmungsöffnung in einem größeren Abstand zur virtuellen Ebene angeordnet als die Abströmungsöffnung. Die Zu- und Abströmungsöffnung sind in diesem Ausführungsbeispiel von einer die Rotationsachse enthaltenden Schnittebene durchsetzt, d.h. Zu- und Abströmungsöffnungen befinden sich bei seitlicher Betrachtung auf die Probenkammer übereinander. Zudem sind die Zu- und Abströmungsöffnung in der gleichen Halbebene relativ zu einer die Rotationsachse enthaltenden und orthogonal zu der Schnittebene orientierten Trennebene angebracht, d.h. die Zu- und Abströmungsöffnung befinden sich jeweils auf der gleichen Probenkammerwandseite. Innerhalb der Probenkammer ist ein Strömungsleitmittel angeordnet, das dafür sorgt, dass die in den von der Probenkammer umfassten Raumbereich eintretende Zuluft oder Gaszuführung in einen kuppennahen Unterbereich der Probenkammer gelangt und durch die konkave Krümmung der Probenkammerinnenwand in Richtung zur Pflanzenblattoberfläche umgelenkt wird, um diese möglichst raumnah zu überströmen, bevor die Gasströmung die Probenkammer über die Abströmöffnung verlässt. Einzelheiten zu dieser Ausführungsform sind im Weiteren unter Bezugnahme auf die Figuren beschrieben.

Der lösungsgemäße Gasprobensammler eröffnet aufgrund des geringen Gewichtes, der geringen Herstellungskosten sowie der sehr guten Materialverfügbarkeit Gasprobensammler in einer großen Anzahl an Pflanzenblätter einer Pflanze zu befestigen, so dass eine Vielzahl von Blättern bspw. eines Baumes oder Strauches in unterschiedlichen Bereichen bspw. einer Baumkrone simultan untersucht werden können, um auf diese Weise belastbare Aussagen über die Photosynthese-Aktivitäten in unterschiedlichen Bereichen der Baumkrone zu erhalten.

Die Anwendung eines Gasprobensammlers erfolgt in der nachfolgenden Verfahrensweise: Zunächst wird die Probenkammer an der Oberfläche, vorzugsweise an der Unterseite eines Pflanzenblattes angebracht, wodurch ein Raumbereich geschaffen wird, der vom Pflanzenblatt und ansonsten von der Probenkammer begrenzt wird. In diesen Raumbereich wird ein Trägergas einer bestimmten Qualität eingeleitet, das innerhalb der Probenkammer und insbesondere durch Kontakt mit der Pflanzenblattoberseite mit Zusatzstoffen angereichert wird, die durch Gaswechselvorgängen im Pflanzenblatt gebildet werden. Das mit diesen Zusatzstoffen angereicherte Trägergas wird nachfolgend aus der Probenkammer abgeleitet, gesammelt und nachfolgend analysiert.

### Kurze Beschreibung der Erfindung

Die Erfindung wird nachstehend ohne Beschränkung des allgemeinen Erfindungsgedankens anhand von Ausführungsbeispielen unter Bezugnahme auf die Zeichnungen exemplarisch beschrieben. Es zeigen:
- Fig. 1a: Explosionsdarstellung einer lösungsgemäß ausgebildeten Probenkammer,
- Fig. 1b: Querschnitt durch die Probenkammer nach Figur 1a,
- Fig. 2: Draufsicht auf ein Strömungsleitmittel,
- Fig. 3: alternatives Ausführungsbeispiel für eine Probenkammer sowie
- Fig. 4: Diagrammdarstellung zur Untersuchung der Assimilationsrate bei Blättern mit lösungsgemäßem Gasprobensammler

### Wege zur Ausführung der Erfindung, gewerbliche Verwendbarkeit

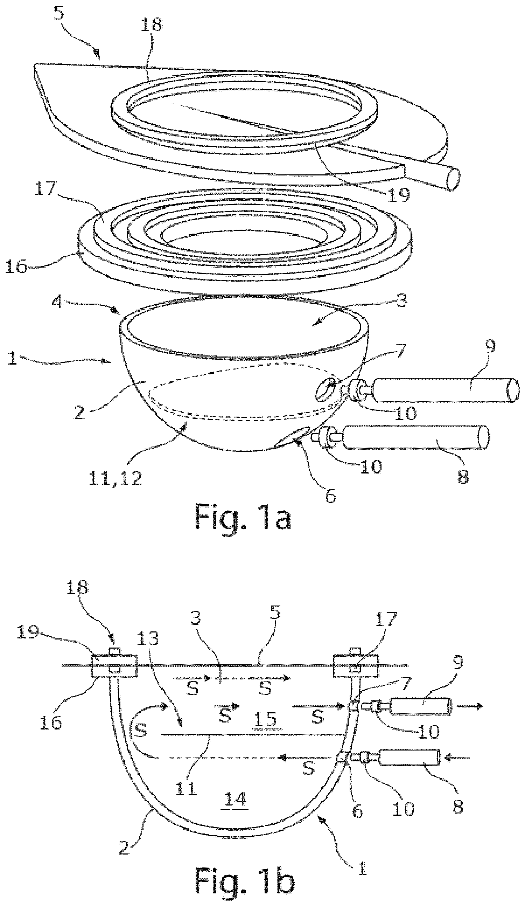

Fig. 1a zeigt eine Explosionsdarstellung eines lösungsgemäß ausgebildeten Gasprobensammlers. Fig. 1b zeigt einen Querschnitt durch den Gasprobensammler. Der Gasprobensammler weist eine Probenkammer 1 auf, die eine vorzugsweise lichttransparente, schalen- bzw. kuppenförmige Probenkammerwand 2 besitzt, die einen inneren Raumbereich 3 begrenzt, der über den im dargestellten Fall kreisringförmig ausgebildeten Probenkammerrand 4 frei zugänglich ist. Die Probenkammerwand 2 ist aus lichttransparentem Material, vorzugsweise aus leichtgewichtigem PET gefertigt und besitzt ein Eigengewicht von nur wenigen Gramm. Form und Größe der Probenkammer 1 richtet sich nach der Form und Größe des jeweiligen Pflanzenblattes 5. Nicht notwendigerweise ist der Probenkammerrand 4 kreisringförmig ausgebildet ist. Beispielsweise ist es denkbar, die Form des Probenkammerrandes 4 an die Blattform anzupassen, so beispielsweise an die Unterseite eines Ahorn-Blattes, bei dem sich eine "fingerförmige" Umrisskontur für den Probenkammerrand anböte.

In der Probenkammerwand 2 sind zwei weitere Öffnungen eingebracht, eine Zuströmöffnung 6 sowie eine Abströmöffnung 7, die jeweils für einen fluiddichten Leitungsanschluss, nämlich dem Anschluss einer Gaszuführungsleitung 8 und einer Gasabführungsleitung 9 geeignet ausgebildet sind. Vorzugsweise befinden sich an der Zu- und Abströmungsöffnung 6, 7 jeweils ein fluiddynamischer Adapter 10, über den eine lösbar fluiddichte Anbringung der Gaszu- und Gasabführungsleitungen 8, 9 möglich ist.

Im dargestellten Ausführungsbeispiel befinden sich die Zuströmöffnung 6 sowie Abströmöffnung 7 längs einer gemeinsamen Schnittebene, die eine durch den kreisringförmigen Probenkammerrand 4 gebildete virtuelle Ebene orthogonal schneidet. Zudem befinden sich beide Öffnungen 6, 7 auf der gleichen Halbebene relativ zu einer Ebene, die die Schnittebene sowie die virtuelle Ebene orthogonal schneidet.

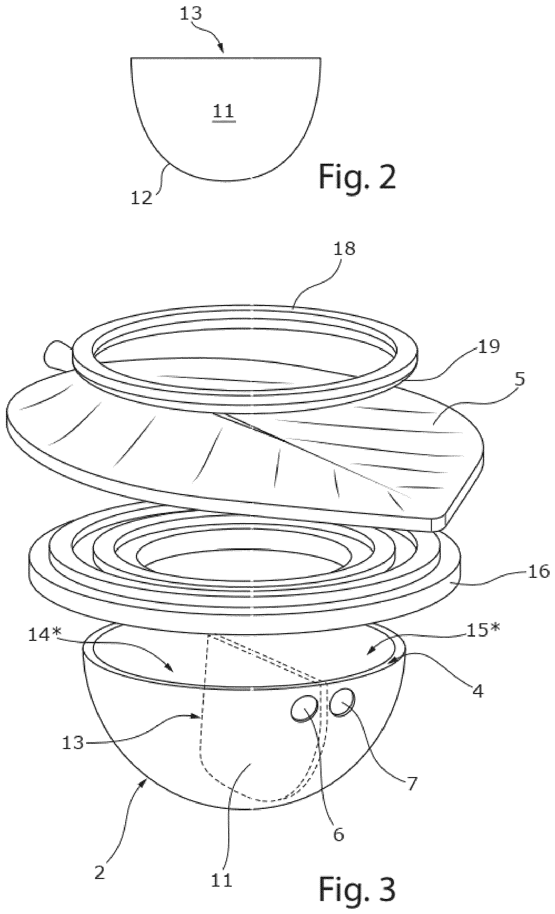

Zwischen beiden Öffnungen 6, 7 ist ein plattenförmiges Strömungsleitmittel 11 angebracht, siehe Figur 2, das mit dessen kreisförmig ausgebildeten Teilumfangsrand 12 mit der Innenwand der Probenkammerwand 2 fluiddicht verbunden ist. Die geradlinig ausgebildete Seitenkante 13 des Strömungsleitmittels 11 ist von der Probenkammerwand 2 beabstandet und schafft somit einen Verbindungsbereich zwischen einem ersten Unterbereich 14 des Raumbereiches 3, in den die Zuströmungsöffnung 6 mündet zu einem zweiten Unterbereich 15, in den die Abströmungsöffnung 7 mündet.

Ferner umfasst der Gasprobensammler ein Dichtmittel 16 in Form einer elastischen ringförmig ausgebildeten Flächendichtung, die zwischen der Probenkammer 1 und dem Pflanzenblatt 5 angeordnet ist. Bei dem Pflanzenblatt 5 handelt es sich um ein "lebendes" Pflanzenblatt, d.h. das Pflanzenblatt 5 ist mit der Pflanze, beispielsweise einem Baum, verbunden. Dadurch ist sichergestellt, dass das Pflanzenblatt äußeren Umwelteinflüssen ausgesetzt ist und entsprechend Photosynthese-aktiv ist. Die elastische Flächendichtung 16 verfügt über eine der Probenkammer 1 zugewandte Oberfläche, in der vorzugsweise (nicht dargestellt) eine auf die Form und Größe des Probenkammerrandes 4 entsprechend gegenkonturierte nutförmige Ausnehmung eingebracht ist, in die die Probenkammer 1 über ihren Probenkammerrand 4 fluiddicht und dauerhaft fügbar ist.

Vorzugsweise eignet sich zur Ausbeildung der elastischen, ringförmig ausgebildeten Flächendichtung Silikon. Es hat sich zudem gezeigt, dass sich die Transmission der Silikondichtung von mindestens 70 % auf 80 % erhöht, wenn z. B. eine 10 nm dicke Aluminiumoxidbeschichtung aufgebracht wird. Die Beschichtung kann an die Umgebungsbedingungen angepasst werden und in Dicke und Material variieren.

Zum Zwecke einer möglichst schonenden, wieder-lösbaren Befestigung der Probenkammer 1 über die elastische Flächendichtung 16 an der Unterseite des Pflanzenblattes 5, ist zusätzlich ein Fügemittel vorgesehen, das eine erste Magneteinheit 17 sowie eine zweite Magneteinheit 18 umfasst. Beide Magneteinheiten 17, 18 sind ringförmig gleich dimensioniert ausgebildet. Die erste Magneteinheit 17 ist innerhalb der elastischen Flächendichtung 16 integriert oder in dieser lösbar fest gefügt und kommt mit dieser an der Unterseite des Pflanzenblattes 5 zur Anlage. Die zweite Magneteinheit 18 stellt eine separat handhabbare Einheit dar und kommt auf der Oberseite des Pflanzenblattes 5 zur Anlage. Beide Magneteinheiten 17 und 18 sind vorzugsweise aus einem lichttransparenten Material gefertigt. Zwischen der zweiten Magneteinheit 18 und der Oberseite des Pflanzenblattes 5 ist vorzugsweise aus Gründen der Blattschonung ebenfalls ein lichttransparentes, elastisches Schichtelement 19 angeordnet. Messungen haben zudem ergeben, dass durch Vorsehen eines lichttransparenten, elastischen Schichtelementes 19 zwischen der zweiten Magneteinheit 18 und der Oberseite des Pflanzenblattes 5 mindestens 8% des senkrecht auf das Pflanzenblatt 5 einfallenden Lichts für den für die Photosynthese relevanten Wellenlängenbereich von 400 - 700 nm durchgelassen wird. Durch die magnetischen Anziehungskräfte zwischen den beiden Magneteinheit 17, 18 haftet der Gasprobensammler an der Unterseite des Pflanzenblattes 5 an.

Das plattenförmig ausgebildete Strömungsleitmittel 11 lenkt die durch die Zuströmöffung 7 in den Unterbereich 14 der Probenkammer 1 eintretende Trägergasströmung S gemeinsam mit der konkav geformten Probenkammerinnenwand 2 im Bereich ihrer Seitenkante 13 in den Unterbereich 15 um, in dem die Trägergasströmung S die Unterseite des Pflanzenblattes 5 überströmt und dort mit aus dem Pflanzenblatt 5 austretenden Gasbestandteilen angereichert wird. Das mit den vom Pflanzenblatt 5 emittierten Gasbestandteilen angereicherte Trägergas S gelangt stromab durch die Abströmungsöffnung 7 und die daran angeschlossene Gasabführungsleitung 9 zum Zwecke einer Gassammlung und nachfolgenden Gasanalyse

Figur 3 zeigt eine alternative Ausführungsvariante zur Gestaltung der Probenkammer 1, die gleichsam wie im Fallbeispiel gemäß Figur 1a, b eine halbkugelförmig gestaltete Probenkammerwand 2 besitzt, jedoch über eine unterschiedliche Anordnung der Zuströmöffnung 6 und Abströmöffnung 7 verfügt. Im dargestellten Fall ist das plattenförmige Strömungsleitmittel 11 orthogonal zur Unterseite des Pflanzenblattes 5 orientiert und nicht wie im Fall gem. Fig. 1a, b parallel dazu. Auf diese Weise unterteilt das Strömungsleitmittel 11 die halbkugelförmig ausgebildete Probenkammer 1 in zwei Kugelviertelsegmentartige Unterbereiche 14*, 15*, wobei auch in diesem Fall das plattenförmig ausgebildete Strömungsleitmittel 11 einen Strömungsübergang zwischen beiden Unterbereichen 14*, 15* im Bereich ihrer Seitenkante 13 ermöglicht, die orthogonal zu den durch den Probenkammerrand 4 aufgespannten virtuellen Ebene orientiert und beabstandet zur Probenkammerinnenwand angeordnet ist.

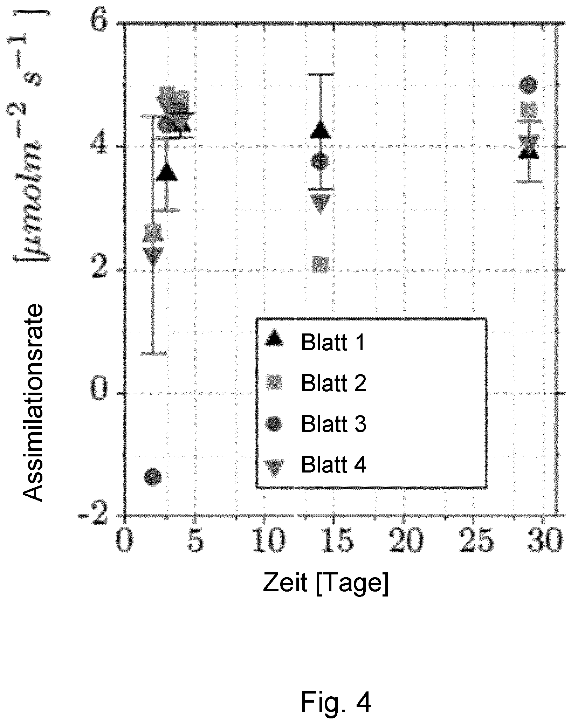

Fig. 4 zeigt das Ergebnis zur Untersuchung der Assimilationsrate [µmol m-2 s-1] nach Anbringung des lösungsgemäßen Gassammlers an vier Blättern einer Buche. Es zeigte sich, dass sich direkt nach Anbringung des Gassammlers an den Blättern die Assimilationsrate stressbedingt reduzierte. In den nachfolgenden Tagen nach der Anbringung erholten sich die Blätter wieder, was sich durch eine Zunahme der Assimilationsrate darstellt. Wäre das hingegen Blatt gestört, bspw. durch Abtrennung vom Ast, würde die Assimilationsrate weiter sinken.

Weitere Vergleichsmessungen mit einer bekannten Messmethode nach Art der eingangs erläuterten Schlauchbeutelmethode nach Haberstroh, die die Erfassung von CO₂, Wasser sowie gängigen VOC, wie bspw. Azeton, Hexenal, Isopren, Azetalhyde, umfassten, zeigten, dass die lösungsgemäßen Gasprobensammler vergleichbare Messergebnisse liefern. In Hinblick auf die Geschwindigkeit der Messwerterfassung sind die dezentral an den einzelnen Blättern angebrachten, lösungsgemäßen Gasprobensammlern der bekannten Messmethode deutlich überlegen, zumal die Gasprobensammler über ein kleineres Messvolumen verfügen, dadurch höhere Durchflussraten und damit eine höhere zeitliche Auflösung erzielen.

### Bezugszeichenliste

- 1: Probenkammer
- 2: Probenkammerwand
- 3: Raumbereich
- 4: Probenkammerrand
- 5: Pflanzenblatt
- 6: Zuströmöffnung
- 7: Abströmöffnung
- 7: Gaszuführungsleitung
- 9: Gasabführungsleitung
- 10: Adapter
- 11: Strömungsleitmittel
- 12: kreisförmiger Teilumfangsrand
- 13: Seitenkante
- 14: erster Unterbereich
- 15: zweiter Unterbereich
- 16: Dichtmittel, elastische Flächendichtung
- 17: erste Magneteinheit
- 18: zweite Magneteinheit
- 19: lichttransparentes Schichtelement

## Patentansprüche

1. Gasprobensammler zum Erfassen von Gaswechselvorgängen an einem Pflanzenblatt (5) mit einer Probenkammer (1),
die Probenkammer (1) eine Probenkammerwand (2) besitzt, in der eine Zu- (6) und eine Abströmungsöffnung (7) angeordnet ist und die einen Raumbereich (3) begrenzt, der über eine, von einem unterbrechungsfrei umlaufenden Probenkammerrand (4) umgebende Probenkammeröffnung frei zugänglich ist, und **dadurch gekennzeichnet, dass** längs des Probenkammerrandes (4) eine Fügeeinheit angebracht ist, die geeignet ausgebildet ist den Probenkammerrand (4) fluiddicht an eine Oberfläche eines Pflanzenblattes (5) derart lösbar fest zu fügen, so dass im gefügten Zustand das Pflanzenblatt (5) den Raumbereich (3) gemeinsam mit der Probenkammerwand (2) begrenzt,
dass die Fügeeinheit ein Fügemittel zur Erzeugung einer Haltekraft, mit der die Probenkammer (1) kraftbeaufschlagt am Pflanzenblatt (5) fügbar ist, sowie ein Dichtmittel (16), zur fluiddichten Fügung der Probenkammer (1) an der Oberseite des Pflanzenblattes (5) aufweist,
dass das Fügemittel wenigstens zwei Magneteinheiten (17, 18) aufweist, von denen eine erste Magneteinheit (17) fest oder lösbar fest mit der Probenkammer (1) verbunden oder verbindbar ist und eine zweite Magneteinheit (18) als separat handhabbare Einheit ausgebildet ist,
dass die die Probenkammer (1) und die Fügeeinheit ein Gesamtgewicht zwischen 3 Gramm und 50 Gramm besitzen und eine Form und Größe besitzen, so dass die Probenkammer (1) selbsttragend an ein mit einer Pflanze verbundenes, lebendes Pflanzenblatt (5) dauerhaft lösbar fluiddicht fügbar ist.

2. Gasprobensammler nach Anspruch 1,
**dadurch gekennzeichnet, dass** die Zuströmungsöffnung (6) mit einer Gaszuführungsleitung (8) und die Abströmungsöffnung (7) mit einer Gasabführungsleitung (9) fluiddicht verbunden oder verbindbar ist.

3. Gasprobensammler nach Anspruch 1 oder 2,
**dadurch gekennzeichnet, dass** die Probenkammer (1) zumindest bereichsweise eine dem Raumbereich (3) zugewandte konkave Probenkammerwandoberfläche besitzt.

4. Gasprobensammler nach einem der Ansprüche 1 bis 3,
**dadurch gekennzeichnet, dass** die Probenkammerwand (2) den Probenkammerrand (4) kuppelförmig überragt.

5. Gasprobensammler nach einem der Ansprüche 1 bis 4,
**dadurch gekennzeichnet, dass** das Dichtmittel (16) eine elastische Flächendichtung aufweist, die sich zumindest im gefügten Zustand längs des Probenkammerrandes (4) unterbrechungsfrei erstreckt und eine mit der Probenkammeröffnung überlappende Öffnung besitzt.

6. Gasprobensammler nach Anspruch 5,
**dadurch gekennzeichnet, dass** die erste Magneteinheit (17) in die elastische Flächendichtung (16) gefügt oder fügbar ist, und dass
der Probenkammerrand (4) in die elastische Flächendichtung (16) fluiddicht gefügt oder fügbar ist.

7. Gasprobensammler nach einem der Ansprüche 1 bis 6,
**dadurch gekennzeichnet, dass** die zweite Magneteinheit (18) in Form und Größe der ersten Magneteinheit (17) entspricht und im gefügten Zustand deckungleich zur ersten Magneteinheit (17) an einer der Oberfläche des Pflanzenblattes (5) gegenüberliegenden Oberfläche zu Anlage bringbar ist.

8. Gasprobensammler nach einem der Ansprüche 1 bis 7,
**dadurch gekennzeichnet, dass** der Probenkammerrand (4) eine virtuelle Ebene aufspannt, über die die Probenkammer (1) rotationssymmetrisch zu einer orthogonal zur virtuellen Ebene orientierten Rotationsachse erhaben ausgebildet ist.

9. Gasprobensammler nach Anspruch 8,
**dadurch gekennzeichnet, dass** die Zuströmungsöffnung (6) in einem größeren Abstand zur virtuellen Ebene angeordnet ist als die Abströmungsöffnung (7),
dass die Zu- und Abströmungsöffnung (6, 7) von einer die Rotationsachse enthaltenden Schnittebene durchsetzt sind, und
dass die Zu- und Abströmungsöffnung (6, 7) in einer gleichen Halbebene relativ zu einer die Rotationsachse enthaltenden und orthogonal zur Schnittebene orientierten Trennebene angeordnet sind.

10. Gasprobensammler nach Anspruch 9,
**dadurch gekennzeichnet, dass** zwischen der Zu- und Abströmungsöffnung (6, 7) ein Strömungsleitmittel (11) angeordnet ist, das den Raumbereich (3) in zwei Unterbereiche (14, 15) räumlich separiert, die über einen lokalen Verbindungsbereich fluidisch miteinander verbunden sind, der in einer zur Halbebene, in der die Zu- und Abströmungsöffnung (6, 7) angeordnet sind, gegenüberliegenden Halbebene angeordnet ist.

11. Gasprobensammler nach einem der Ansprüche 1 bis 10,
**dadurch gekennzeichnet, dass** die Probenkammerwand (2) aus einem lichttransparenten Material gefertigt ist.

12. Gasprobensammler nach einem der Ansprüche 1 bis 11,
**dadurch gekennzeichnet, dass** der Probenkammerrand einem Kreisring entspricht mit einem inneren Ringdurchmesser D mit 10 mm ≤ D ≤ 250 mm, vorzugsweise D = 30 mm ± 10 mm.

13. Gasprobensammler nach einem der Ansprüche 2 bis 12,
**dadurch gekennzeichnet, dass** die mit der Probenkammer (1) verbundene Gaszuführungs- und Gasabführungsleitung (8, 9) zum selbsttragenden, dauerhaften Anbringen an einen Ast der Pflanze geeignet ausgebildet ist, mit dem das mit der Probenkammer (1) versehene Pflanzenblatt (5) verbunden ist.

14. Verfahren zur Gasprobensammlung für ein Erfassen von Gaswechselvorgängen an einem Pflanzenblatt unter Verwendung eines Gasprobensammlers nach einem der Ansprüche 1 bis 13
, **gekennzeichnet durch** folgende Verfahrensschritte:
- Ausbilden eines einseitig von einer Oberfläche eines mit einer Pflanze verbundenen Pflanzenblattes (5) begrenzten Raumbereiches (3), der ansonsten von einer Probenkammer (1) begrenzt wird,
- Einleiten eines Trägergases in den Raumbereich (3),
- Ausleiten des durch Gaswechselvorgänge im Pflanzenblatt mit Zusatzstoffen angereicherten Trägergases.
- Sammeln und/oder Analysieren angereicherten Trägergases.

15. Verfahren nach Anspruch 14,
**dadurch gekennzeichnet, dass** das Einleiten des Trägergases in einen ersten Unterbereich (14) des Raumbereiches (3) erfolgt, der von der Oberfläche des Pflanzenblattes (5) separiert ist,
dass das Ausleiten des angereicherten Trägergases aus einem zweiten Unterbereich (15) des Raumbereiches (3) erfolgt, der einseitig von der Oberfläche des Pflanzenblattes (5) begrenzt ist, und
dass die Unterbereiche (14, 15) derart miteinander verbunden und durchströmt werden, dass das Trägergas und das angereicherte Trägergas in Gegenströmung zueinander orientiert sind.

## Claims

1. A gas sample collector with a sample chamber (1), for measuring gas exchange processes on a plant leaf (5),
the sample chamber (1) having a sample chamber wall (2) in which an inflow opening (6) and an outflow opening (7) are disposed, the sample chamber delimiting a spatial region (3) which is freely accessible via a sample chamber opening which is surrounded by a continuous circumferential sample chamber edge (4), and
**characterized in that** along the sample chamber edge (4), a joining unit is attached which is appropriately constructed to releasably and firmly join the sample chamber edge (4) to a surface of a plant leaf (5) in a fluid-tight manner such that, in the joined state, the plant leaf (5) together with the sample chamber wall (2) define the spatial region (3),
**in that** the joining unit has a joining means for generating a retaining force, with which the sample chamber (1) can be joined to the plant leaf (5) under force, as well as a sealing means (16) for joining the sample chamber (1) to the upper side of the plant leaf (5) in a fluid-tight manner,
**in that** the joining means has at least two magnetic units (17, 18), of which a first magnetic unit (17) is firmly or releasably connected or connectable to the sample chamber (1) and a second magnetic unit (18) is constructed as a separately manageable unit,
**in that** the sample chamber (1) and the joining unit have a total weight of between 3 grams and 50 grams and have a shape and size such that the sample chamber (1) can be permanently and releasably joined in a fluid-tight manner and in a self-supporting manner to a living plant leaf (5) connected to a plant.

2. The gas sample collector as claimed in claim 1,
**characterized in that** the inflow opening (6) is connected or connectable in a fluid-tight manner to a gas supply line (8) and the outflow opening (7) is connected or connectable in a fluid-tight manner to a gas discharge line (9).

3. The gas sample collector as claimed in claim 1 or claim 2,
**characterized in that** the sample chamber (1) has a sample chamber wall surface which is concave towards the spatial region (3), at least in regions.

4. The gas sample collector as claimed in one of claims 1 to 3,
**characterized in that** the sample chamber wall (2) protrudes beyond the sample chamber edge (4) in a dome shape.

5. The gas sample collector as claimed in one of claims 1 to 4,
**characterized in that** the sealing means (16) has an elastic surface seal which extends continuously along the sample chamber edge (4) at least in the joined state and has an opening which overlaps with the sample chamber opening.

6. The gas sample collector as claimed in claim 5,
**characterized in that** the first magnetic unit (17) is or can be joined to the elastic surface seal (16), and **in that**
the sample chamber edge (4) is or can be joined to the elastic surface seal (16) in a fluid-tight manner.

7. The gas sample collector as claimed in one of claims 1 to 6,
**characterized in that** the second magnetic unit (18) corresponds in shape and size to the first magnetic unit (17) and, in the joined state, can be brought into abutment in a position which is aligned with the first magnetic unit (17) at a surface which is opposite the surface of the plant leaf (5).

8. The gas sample collector as claimed in one of claims 1 to 7,
**characterized in that** the sample chamber edge (4) defines a virtual plane via which the sample chamber (1) is constructed in a rotationally symmetrical manner with respect to an axis of rotation which is orientated orthogonally with respect to the virtual plane.

9. The gas sample collector as claimed in claim 8,
**characterized in that** the inflow opening (6) is disposed at a greater distance from the virtual plane than the outflow opening (7),
**in that** a sectional plane containing the axis of rotation passes through the inflow opening and outflow opening (6, 7), and
**in that** the inflow opening and outflow opening (6, 7) are disposed in the same half-plane relative to a separating plane containing the axis of rotation and orientated orthogonally with respect to the sectional plane.

10. The gas sample collector as claimed in claim 9,
**characterized in that** a flow guide means (11) is disposed between the inflow opening and outflow opening (6, 7) which spatially separates the spatial region (3) into two sub-regions (14, 15) which are fluidically connected to each other via a local connecting region which is disposed in a half-plane which is opposite the half-plane in which the inflow opening and outflow opening (6, 7) are disposed.

11. The gas sample collector as claimed in one of claims 1 to 10,
**characterized in that** the sample chamber wall (2) is produced from a material which is transparent to light.

12. The gas sample collector as claimed in one of claims 1 to 11,
**characterized in that** the sample chamber edge corresponds to a circular ring with an internal ring diameter D with 10 mm ≤ D ≤ 250 mm, preferably D = 30 mm ± 10 mm.

13. The gas sample collector as claimed in one of claims 2 to 12,
**characterized in that** the gas supply line and gas discharge line (8, 9) connected to the sample chamber (1) are constructed for self-supporting, permanent attachment to a branch of the plant to which the plant leaf (5) provided with the sample chamber (1) is connected.

14. A method for collecting gas samples for measuring gas exchange processes on a plant leaf using a gas sample collector as claimed in one of claims 1 to 13,
**characterized by** the following steps of the method:
- constructing a spatial region (3) which is delimited on one side by a surface of a plant leaf (5) connected to a plant, the spatial region being otherwise delimited by a sample chamber (1),
- introducing a carrier gas into the spatial region (3),
- recovering the carrier gas which is enriched with additional substances by gas exchange processes in the plant leaf,
- collecting and/or analysing enriched carrier gas.

15. The method as claimed in claim 14,
**characterized in that** the carrier gas is introduced into a first sub-region (14) of the spatial region (3) which is separated from the surface of the plant leaf (5),
**in that** the enriched carrier gas is recovered from a second sub-region (15) of the spatial region (3) which is delimited on one side by the surface of the plant leaf (5), and
**in that** the sub-regions (14, 15) are connected to each other and perfused in a manner such that the carrier gas and the enriched carrier gas are orientated as a counter-current with respect to each other.

## Revendications

1. Collecteur d'échantillons gazeux, destiné à la détection de processus d'échanges gazeux sur une feuille (5) de plante, pourvu d'une chambre d'échantillonnage (1),
la chambre d'échantillonnage (1) présente une paroi (2) de chambre d'échantillonnage, dans laquelle est placé un orifice d'affluence (6) et d'échappement (7) et qui délimite une zone d'espace (3) qui est librement accessible par l'intermédiaire d'un orifice de la chambre d'échantillonnage entouré par un bord (4) de chambre d'échantillonnage périphérique sans interruption et
**caractérisé en ce que** le long du bord (4) de chambre d'échantillonnage est montée une unité d'assemblage qui est conçue de sorte à être apte à assembler le bord (4) de chambre d'échantillonnage de manière étanche aux fluides sur une surface d'une feuille (5) de plante de manière fixe amovible, de telle sorte que dans l'état assemblé, la feuille (5) de plante délimite la zone d'espace (3) conjointement avec la paroi (2) de chambre d'échantillonnage,
**en ce que** l'unité d'assemblage comporte un moyen d'assemblage, destiné à générer une force de maintien, à l'aide de laquelle, soumise à une force, la chambre d'échantillonnage (1) est susceptible d'être assemblée sur la feuille (5) de plante, ainsi qu'un moyen d'étanchéité (16), destiné à l'assemblage étanche aux fluides de la chambre d'échantillonnage (1) sur la face supérieure de la feuille (5) de plante,
**en ce que** le moyen d'assemblage comporte au moins deux unités magnétiques (17, 18), dont une première unité magnétique (17) est reliée ou susceptible d'être reliée de manière fixe ou fixe amovible avec la chambre d'échantillonnage (1) et une deuxième unité magnétique (18) est conçue sous la forme d'une unité manipulable séparément,
**en ce que** la chambre d'échantillonnage (1) et l'unité d'assemblage présentent un poids total compris entre 3 grammes et 50 grammes et présentent une forme et une dimension telles, que la chambre d'échantillonnage (1) soit susceptible d'être assemblée de manière étanche aux fluides, en étant amovible en permanence, de manière autoportante sur une feuille (5) de plante vivante, reliée avec une plante.

2. Collecteur d'échantillons gazeux selon la revendication 1,
**caractérisé en ce que** l'orifice d'affluence (6) est relié ou susceptible d'être relié de manière étanche aux fluides avec une conduite d'alimentation (8) de gaz et l'orifice d'échappement (7) est relié ou susceptible d'être relié de manière étanche aux fluides à une conduite d'évacuation (9) de gaz.

3. Collecteur d'échantillons gazeux selon la revendication 1 ou 2,
**caractérisé en ce que** la chambre d'échantillonnage (1) présente au moins par endroits une surface de paroi de chambre d'échantillonnage concave qui fait face à la zone d'espace (3).

4. Collecteur d'échantillons gazeux selon l'une quelconque des revendications 1 à 3,
**caractérisé en ce que** la paroi (2) de chambre d'échantillonnage saillit en forme de coupole au-delà du bord (4) de chambre d'échantillonnage.

5. Collecteur d'échantillons gazeux selon l'une quelconque des revendications 1 à 4,
**caractérisé en ce que** le moyen d'étanchéité (16) comporte un joint de surface élastique, qui au moins dans l'état assemblé s'étend sans interruption le long du bord (4) de chambre d'échantillonnage et présente un orifice qui se chevauche avec l'orifice de la chambre d'échantillonnage.

6. Collecteur d'échantillons gazeux selon la revendication 5,
**caractérisé en ce que** la première unité magnétique (17) est assemblée ou susceptible d'être assemblée dans le joint de surface (16) élastique et **en ce que**
le bord (4) de chambre d'échantillonnage est assemblé ou susceptible d'être assemblé de manière étanche aux fluides dans le joint de surface (16) élastique.

7. Collecteur d'échantillons gazeux selon l'une quelconque des revendications 1 à 6,
**caractérisé en ce que** la deuxième unité magnétique (18) correspond au niveau de la forme et des dimensions à la première unité magnétique (17) et à l'état assemblé, est susceptible d'être appliquée en coïncidence avec la première unité magnétique (17) sur une surface opposée à la surface de la feuille (5) de plante.

8. Collecteur d'échantillons gazeux selon l'une quelconque des revendications 1 à 7,
**caractérisé en ce que** le bord (4) de chambre d'échantillonnage définit un plan virtuel au-delà duquel la chambre d'échantillonnage (1) est conçue en étant élevée de manière symétrique en rotation par rapport à un axe de rotation orienté à l'orthogonale du plan virtuel.

9. Collecteur d'échantillons gazeux selon la revendication 8,
**caractérisé en ce que** l'orifice d'affluence (6) est placé par rapport au plan virtuel avec un écart supérieur à celui de l'orifice d'échappement (7),
**en ce que** l'orifice d'affluence et d'échappement (6, 7) sont traversés par un plan de coupe contenant l'axe de rotation et
**en ce que** l'orifice d'affluence et d'échappement (6, 7) sont placés dans un même demi-plan par rapport à un plan de séparation contenant l'axe de rotation et orienté à l'orthogonale du plan de coupe.

10. Collecteur d'échantillons gazeux selon la revendication 9,
**caractérisé en ce qu'**entre l'orifice d'affluence et d'échappement (6, 7) est placé un moyen de guidage (11) de flux, qui sépare dans l'espace la zone d'espace (3) en deux sous-zones (14, 15) qui sont fluidiquement reliées l'une avec l'autre par l'intermédiaire d'une zone de liaison locale, qui est placée dans un demi-plan opposé au demi-plan dans lequel sont placés l'orifice d'affluence et d'échappement (6, 7).

11. Collecteur d'échantillons gazeux selon l'une quelconque des revendications 1 à 10,
**caractérisé en ce que** la paroi (2) de chambre d'échantillonnage est fabriquée dans une matière transparente à la lumière.

12. Collecteur d'échantillons gazeux selon l'une quelconque des revendications 1 à 11,
**caractérisé en ce que** le bord de chambre d'échantillonnage correspond à un anneau circulaire, d'un diamètre intérieur de l'anneau D de 10 mm ≤ D ≤ 250 mm, de préférence de D = 30mm ± 10mm.

13. Collecteur d'échantillons gazeux selon l'une quelconque des revendications 2 à 12,
**caractérisé en ce que** la conduite d'alimentation de gaz et d'évacuation de gaz (8, 9) reliée avec la chambre d'échantillonnage (1) est conçue pour être apte à être montée en permanence, de manière autoportante sur une branche de la plante avec laquelle est reliée la feuille (5) de plante munie de la chambre d'échantillonnage (1).

14. Procédé, destiné à collecter des échantillons gazeux pour une détection de processus d'échanges gazeux sur une feuille de plante, en utilisant un collecteur d'échantillons gazeux selon l'une quelconque des revendications 1 à 13,
**caractérisé par** les étapes de procédé suivantes, consistant à :
- constituer une zone d'espace (3), délimitée d'un côté par une surface d'une feuille (5) de plante reliée avec une plante, que l'on délimite par ailleurs par une chambre d'échantillonnage (1),
- introduire un gaz porteur dans la zone d'espace (3) ,
- extraire le gaz porteur enrichi en additifs par le processus d'échanges gazeux dans la feuille de plante,
- collecter et / ou analyser le gaz porteur enrichi.

15. Procédé selon la revendication 14,
**caractérisé en ce que** l'introduction du gaz porteur s'effectue dans une première sous-zone (14) de la zone d'espace (3) qui est séparée de la surface de la feuille (5) de plante,
**en ce que** l'extraction du gaz porteur enrichi s'effectue à partir d'une deuxième sous-zone (15) de la zone d'espace (3), qui est délimitée d'un côté par la surface de la feuille (5) de plante et
**en ce que** les sous-zones (14, 15) sont reliées l'une avec l'autre et irriguées de telle sorte que le gaz porteur et le gaz porteur enrichi soient orientés à contre-courant l'un de l'autre.
